# FASCICULE DE BREVET EUROPEEN

(11) **EP 2 200 986 B1**
(45) Date de publication et mention de la délivrance du brevet: **08.08.2012**
(21) Numéro de dépôt: 08839412.7
(22) Date de dépôt: 17.10.2008
(51) Int. Cl.: C07D 215/12, C07F 9/50

(54) **Composés tridentates chiraux, complexes organométalliques correspondants, procédé d'obtention et utilisation comme ligands en catalyse asymétrique de tels composés et complexes**
Chirale Tridentatverbindungen, entsprechende Organometallkomplexe, Verfahren zu ihrer Herstellung sowie Verwendung dieser Verbindungen und Komplexe als Liganden in der asymmetrischen Katalyse
Chiral tridentate compounds, corresponding organometal complexes, method for preparing same and use of said compounds and complexes as ligands in asymmetrical catalysis

(30) Priorité: 18.10.2007 FR 0758420
(43) Date de publication de la demande: 30.06.2010
(73) Titulaire: Ecole Nationale Superieure de Chimie de Rennes, 35700 Rennes Cedex (FR); CNRS (Centre National de La Recherche Scientifique), 75794 Paris Cedex 16 (FR)
(72) Inventeur: MAUDUIT, Marc, F-35500 Vitre (FR); RIX, Diane, F-35700 Rennes (FR); CREVISY, Christophe, F-35131 Pont-pean (FR); WENCEL, Joanna, F-35510 Cesson-sevigne (FR)
(74) Mandataire: Larcher, Dominique
(86) Numéro de dépôt international: PCT/EP2008/064067
(87) Numéro de publication internationale: WO 2009/050284

(56) Documents cités:
- SYLVIA J. DEGRADO ET AL.: "Efficient Cu-catalyzed asymmetric conjugate additions of alkylzincs to trisubstituted cyclic enones" JOURNAL OF THE AMERICAN CHEMICAL SOCIETY., vol. 124, no. 45, 2002, pages 13362-13363, XP002486895 USAMERICAN CHEMICAL SOCIETY, WASHINGTON, DC.
- HENRI BRUNNER ET AL.: "Asymmetric Catalysis," EUROPEAN JOURNAL OF INORGANIC CHEMISTRY., vol. 1, 1999, pages 51-59, XP002486896 DEWILEY-VCH, WEINHEIM.
- COURTNEY A. LUCHACO-CULLIS ET AL.: "Cu-catalyzed enantioselective conjugate addition..." JOURNAL OF THE AMERICAN CHEMICAL SOCIETY., vol. 124, no. 28, 2002, pages 8192-8193, XP002486897 USAMERICAN CHEMICAL SOCIETY, WASHINGTON, DC.
- HIROTAKE MITZTANI ET AL.: "Cu-catalyzed conjugate additions.." JOURNAL OF THE AMERICAN CHEMICAL SOCIETY., vol. 124, no. 5, 2002, pages 779-781, XP002486898 USAMERICAN CHEMICAL SOCIETY, WASHINGTON, DC.
- NATHAN S. JOSEPHSOHN ET AL.: "Efficient and practical Ag-catalyzed cycloadditions between arylimines and the Danishefsky diene" JOURNAL OF THE AMERICAN CHEMICAL SOCIETY., vol. 125, no. 14, 2003, pages 4018-4019, XP002486899 USAMERICAN CHEMICAL SOCIETY, WASHINGTON, DC.
- EMMA L. CARSWELL ET AL.: "A highly efficient and practical method for catalytic asymmetric vinylogous Mannich reactions" ANGEWANDTE CHEMIE. INTERNATIONAL EDITION., vol. 45, no. 43, 2006, pages 7230-7233, XP002486900 DEVCH VERLAG, WEINHEIM.
- DATABASE BEILSTEIN [Online] BEILSTEIN INSTITUTE FOR ORGANIC CHEMISTRY, FRANKFURT-MAIN, DE; XP002538603 Database accession no. 217087, 217155, 224429, 254214 (BRN) & GIALDI ET AL.: FARMACO., vol. 6, 1951, pages 694-699, ITSOCIETA CHIMICA ITALIANA, PAVIA.
- FEDERICO FERIOLI ET AL.: "Steric effects in enantioselective allylic alkylation catalysed by cationic....." EUROPEAN JOURNAL OF ORGANIC CHEMISTRY., vol. 7, 2005, pages 1416-1426, XP002538661 WILEY-VCH VERLAG, WEINHEIM.

## Description

Le domaine de l'invention est celui de la chimie des catalyseurs.

Plus précisément, la présente invention a pour objet de nouveaux composés tridentates chiraux possédant une architecture de type azométhine.

L'invention concerne également des complexes organométalliques de type métallo-azométhinylate obtenus par complexation de tels composés à divers métaux de transitions, notamment le cuivre, le palladium, le ruthénium, l'iridium et de rhodium.

L'invention concerne également l'utilisation de tels composés ou complexes dans des procédés de catalyse asymétrique mettant en oeuvre divers métaux de transition tels que notamment, mais non exclusivement, les procédés de catalyse énantiosélective d'addition conjuguée, d'hydrogénation, d'activation C-H, d'isomérisation, de couplage C-C et C-N.

La présente invention concerne aussi un procédé de synthèse de tels composés et notamment la synthèse de sels de diphénylphosphinoaryl- et de quinoline- azométhinylates chiraux, à partir d'acides, carboxyliques α- et β- aminés énantiomériquement enrichis et d'aldéhydes aromatiques diversement substitués.
Brunner et al. ont réussi, grâce à un procédé d'alkylation catalysé par le palladium (Eur. J. Inorg. Chem.,1999, 51-59), à obtenir des stéréosiomères intéressants du methohexital, un puissant anesthésique. Grâce à ce procédé, le stéréo-isomère sélectionné peut être administré à des doses plus faibles pour une efficacité identique à celle de la molécule commercialisée. L'équipe de Carlswell et al. ont également mis au point un procédé de catalyse asymétrique stéréosélectif à partir de réactifs carbonés a- et β-aminés (Angewendte Chemie. International Edition, vol.45, n°43, 7230-7233). Ces articles décrivent notamment des ligands tridentates chiraux de type azométhine, utiles pour la catalyse asymétrique.

Un objectif de la présente invention est de proposer de nouveaux ligands tridentates qui puissent être rapidement accessibles à partir d'une source chirale fortement diversifiée et disponible commercialement à faible coût, à savoir des acides carboxyliques a- et p-aminés.

Un autre objectif de l'invention est de décrire de tels nouveaux ligands et les complexes s'y rattachant susceptibles de présenter une excellente réactivité et énantiosélectivité en catalyse asymétrique mettant en jeu divers métaux de transition.

L'invention concerne tout composé de formule (I) : 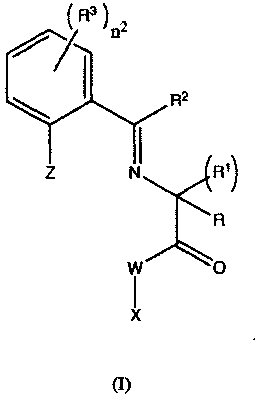 dans laquelle:
W est un atome d'oxygène ou un radical de formule Ni ;
X est un hydrogène, ou un cation alcalin, ou un alkyl en C1 à C8, ou un radical - (CH₂)ₙ³ - C(R⁴)(R⁵)(R⁶) où R⁴, R⁵ et R⁶ sont indépendamment un hydrogène, ou un alkyl en C1 à C8, ou un cycloalkyl en C5 ou C6 ou un aryl ou un naphtyl éventuellement substitués;
n³ = 0 ou 1 ;
n² = 0 à 4 ;
R et R¹ sont indépendamment un alkyl en C1 à C8, ou un cycloalkyl en C5 ou C6 éventuellement substitué, ou un aryl éventuellement substitué, ou un naphtyl éventuellement substitué, ou un radical -(CH₂)ₙ⁴- C(R⁹)(R¹⁰)V où R⁹ et R¹⁰ sont indépendamment un hydrogène, ou un alkyl en C1 à C6, ou un cycloalkyl en C5 ou C6, ou un aryl ;
V est un radical de formule :

   (CH₂)ₙ⁵Y
Y est lui hydrogène, ou un halogène, ou un OR¹¹, ou un SR¹², ou un COOR¹³, ou un NHCOR¹⁴, ou un cycloalkyl en C5 ou C6, ou un aryl éventuellement substitué ;
n⁴ = 0 ou 1 ;
n⁵ = 1 à 6;
R² est un hydrogène, ou un alkyl en C1 à C8, ou un aiyl éventuellement substitué ou un naphtyl ;
R³ est un alkyl en C1 à C6, ou un halogène, ou un OR¹⁵ ;
Z est un radical de formule P(R¹⁶)₂ ou un radical de formule P(O)R¹⁹ ;
R¹¹, R¹², R¹³, R¹⁴, R¹⁵, sont indépendamment un H, ou un alkyl en C1 à C6, ou un cycloalkyl en C5 ou C6 ou un aryl éventuellement substitué ;
R¹⁶, R¹⁷, R¹⁸, R¹⁹, R²⁰, R²¹ sont indépendamment un alkyl en C1 à C6, ou un cycloalkyl en C6, ou un aryl.

Selon une variante, le composé est un diphényl phosphinophényl azométhinylate, tel que notamment un composé de formule **Ia-K**, **Ia-Na** ou **Ib-K**

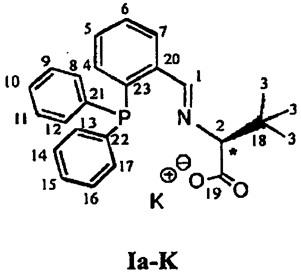

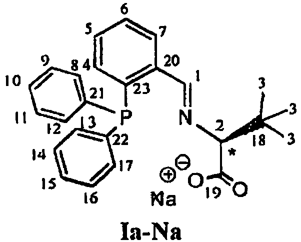

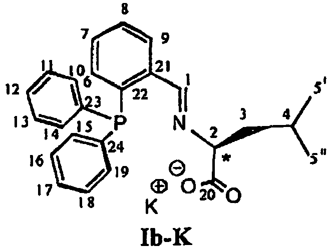

L'invention concerne également tout complexe d'un composé tel que décrit ci-dessus avec au moins un métal choisi dans le groupe constitué par le cuivre, le palladium, le ruthénium, l'iridium et de rhodium, tel que notamment un complexe de formule :

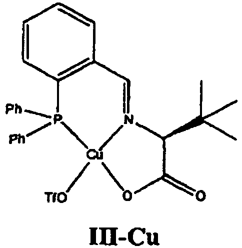

L'invention concerne aussi un procédé de synthèse d'un composé tel que décrit ci-dessus caractérisé en ce qu'il comprend les étapes consistant à :
obtenir un produit de formule A à partir d'un acide carboxylique aminé 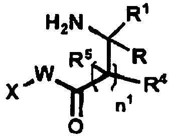
additionner au produit obtenu un aldéhyde ou une cétone choisis dans le groupe des aldéhydes ou des cétones de formule C,

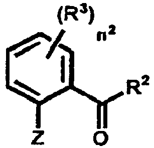

Selon une variante, ledit aldéhyde est choisi dans le groupe constitué par le diphénylphosphine-benzaldéhyde et le quinoline-8 carboxaldéhyde.

Pour les composés décrits ci-dessus de formule **Ia-K** ou **Ia-Na** le procédé selon l'invention met préférentiellement en oeuvre un schéma réactionnel choisi dans le groupe des schémas réactionnels suivants :

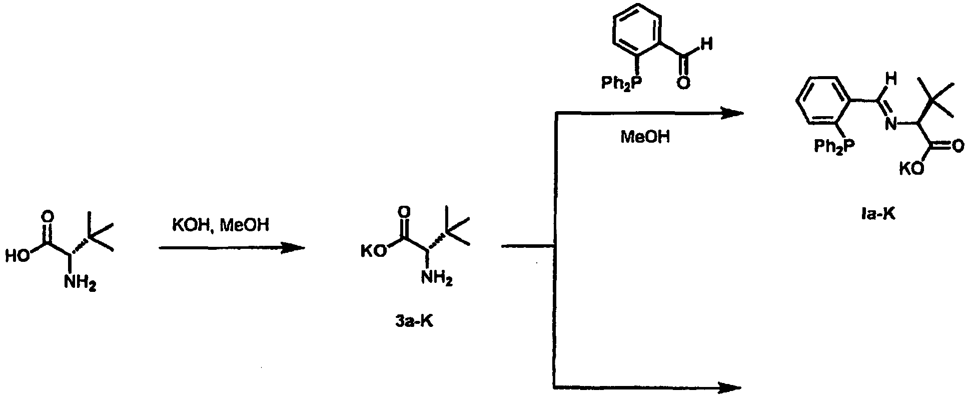

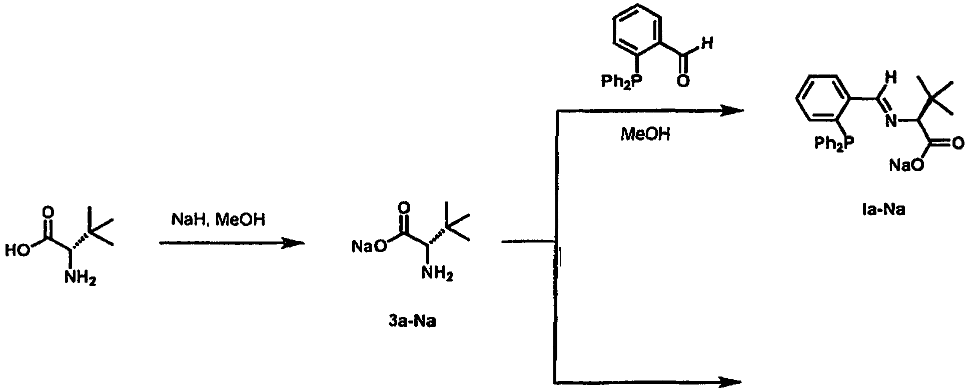

Enfin, la présente invention concerne également toute utilisation d'un composé ou d'un complexe tels que décrits ci-dessus dans le cadre d'un procédé de catalyse asymétrique.

L'invention ainsi que les différents avantages qu'elle présente seront plus facilement compris grâce à la description qui va suivre de différents modes de réalisation de composés et d'un complexe selon celle-ci, ainsi que par la description de l'utilisation de ceux-ci dans différents procédés de catalyse asymétrique.

La synthèse de deux exemples de composés de formule (**I**), à savoir les sels de diphénylphosphinophényl-azométhinylates de sodium et de potassium issus de la tert-leucine (**Ia-Na** et **Ia-K**) est décrite en détail ci-après.

Ces synthèses reprennent les schémas de synthèse représentés ci-dessous :

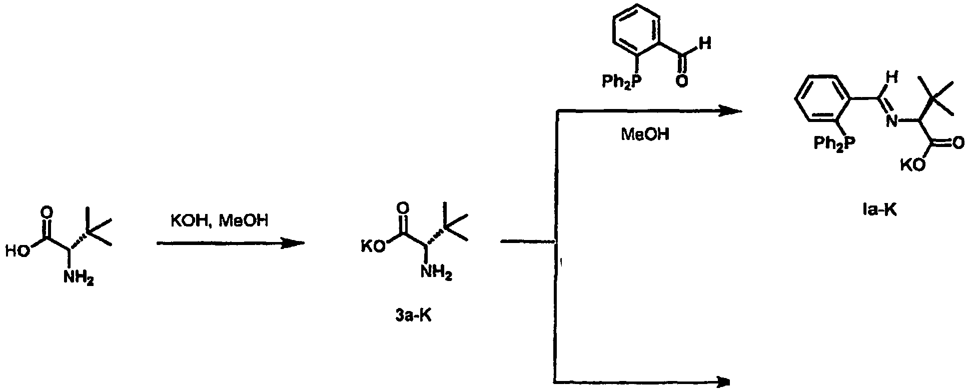

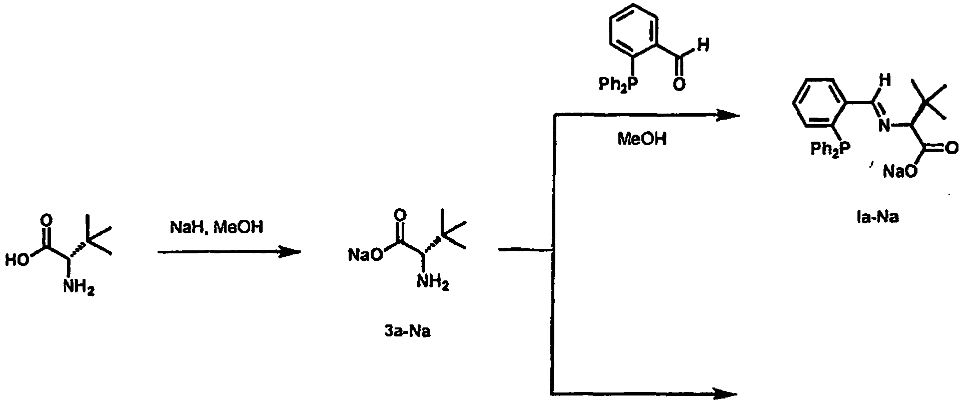

### Synthèse de Ia-K

Dans un bicol équipé d'un réfrigérant, le tamis moléculaire 4 Å est activé. La solution de la *tert*-L(+)-leucine (131 mg, 1 mmol, léq.) et d'hydroxyde de potassium (56 mg, 1 mmol, léq.) dans le méthanol anhydre dégazé (2 mL par mmol) est additionnée. Le *ortho*diphénylphosphine-benzaldéhyde (290 mg, 1 mmol, 1 éq.) est alors ajouté et le mélange est maintenu sous vigoureuse agitation, 3h, à 40°C. Le solvant est alors évaporé. Le *tert-*leucine phosphinoazométhinylate de potassium (**Ia-K**) est obtenu sous forme d'un solide jaunâtre (432 mg, 98%). 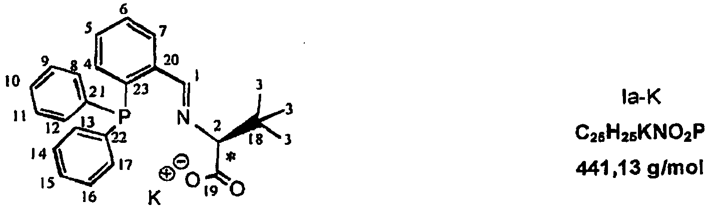 Les caractéristiques RMN de ce composé sont les suivantes :
RMN ¹H : (400 MHz, CD₃DO) δ (ppm) : 8,81 (d, ^{H.P}*J* = 5,3 Hz, 1H, H₁); 8,17 - 8,14 (m, 1H, H₇) ; 7,25 - 7,12 (m, 12H, H₅, H₆, H₈-₁₇) ; 6,73 - 6.69 (m, 1H, H₄) ; 3,28 (s, 1 H, H₂) ; 0,74 (s, 9H, H₃).
RMN ³¹P: (162 MHz, CD₃DO) δ (ppm) : -15,52 (s, P)
RMN ¹³C : (100 MHz, CD₃OD) δ (ppm) : 179,07 (1C, C₁₉) ; 160,15 (1C, C₁); 141,11(1C, C₂₀) ; 138,81 (1C, C₂₁) ; 137,73 (1C, C₂₂) ; 137,40 (1C, C₂₃) ; 135,25 (4C, C_{8,12,13,17}) ; 133,97 (1C, C₄) ; 131,27 (1C, C₇) ; 130,15 - 128,85 (8C, C_{5,6,9,10,11,14,15,16}); 88,64 (1C, C₂) ; 35,29 (1C, C₁₈) ; 27.84 (3C, C₃)

### Synthèse Ia-Na

Un procédé de synthèse similaire à celui utilisé pour la synthèse du composé **Ia-K** a été mis en oeuvre, en utilisant de l'hydroxyde de soude en lieu et place de l'hydroxyde de potassium.

Selon un autre mode de réalisation, la synthèse du composé Ib-K a également été réalisée.

### Synthèse de Ib-K.

Dans un bicol équipé d'un réfrigérant, le tamis moléculaire 4 Å est activé. La solution de la L(+)-leucine (65,5 mg, 0,5 mmol, 1éq.) et d'hydroxyde de potassium (28 mg, 0,5 mmol, 1éq.) dans le méthanol anhydre dégazé (2 mL par mmol) est additionnée. Le *ortho*diphénylphosphine-benzaldéhyde (145 mg, 0,5 mmol, 1 éq.) est alors ajouté et le mélange est maintenu sous vigoureuse agitation, 3h, à 40°C. Le solvant est alors évaporé. L'*iso*-leucine phosphinoazométhinylate de potassium (**Ib-K**) est obtenu sous forme d'un solide jaunâtre (209 mg, 95%).

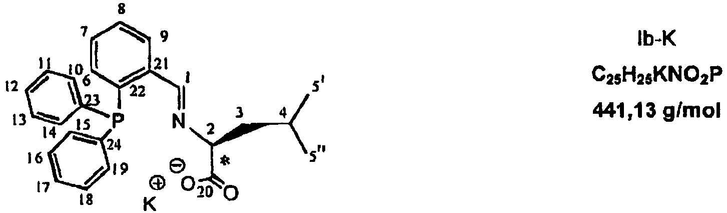

Les caractéristiques RMN de ce composé sont les suivantes :
RMN ¹H : (400 MHz, CD₃DO) δ (ppm) : 8,88 (d, ^{H.P}*J* = 5,5 Hz, 1H, H₁) ; 8,06 - 8,02 (m, 1H, H₉) ; 7,28 - 7,07 (m, 12H, H₇, H₈, H₁₀-₁₉) ; 6,72 - 6,69 (m, 1H, H₆) ; 3,70 (dd² *J* = 4,4 Hz,²*J* = 9,7 Hz, 1H, H₂) ; 1,55 (qd, ³*J* = 4,4 Hz, ²*J* = 9,7 Hz, 2H, H₃) ; 0,89 (m, 1H, Ha) ; 0,62 (d, ³*J* = 6,6 Hz, 3H, H_{5'}) ; 0,51 (d, ³*J* = 6,4 Hz, 3H, H_{5"})
RMN ³¹P : (162 MHz, CD₃DO) δ (ppm) : 14,59 (s, 1P)
RMN¹³C : (100 MHz, CD₃DO) δ (ppm) : 180,90 (1C, C₂₀); 161,59 (1C, C₁); 141,42 (1C, C₂₁); 139,32 (1C, C₂₄) ; 139,13 (1C, C₂₃) ; 138,0 (1C, C₆) ; 137,74 (1C, C₂₂) ; 135,8 (4C, C_{10,14,15,19}) ; 134,33 (1C, C₉) ; 131,87 - 129,13 (8C, C_{7,8,11-13,16-18}) ; 76,94 (1C, C₂) ; 44,54 (1C, C₄) ; 25,7 (1C, C_{5"}) ; 24,3 (1C, C_{5'}) ; 21,9 (1C, C₃)

Dans un mode de réalisation d'un complexe selon l'invention, on a synthétisé à partir du diphénylphosphinophényl-azométhinylate de potassium **Ia-K** le complexe de cuivre (II) **III-Cu** suivant, selon le schéma réactionnel suivant :

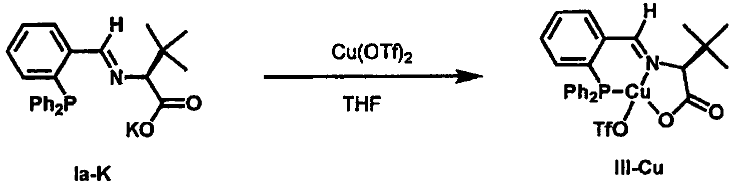

### Synthèse du composé de formule III-Cu

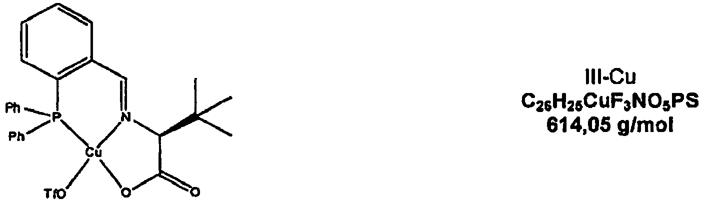

Le sel de *tert*-leucine phosphinoazométhinylate de potassium (100 mg, 0,23 mmol, 1 2q.) et le bis triflate de cuivre Cu(OTf)₂ (114 mg, 0,23 mmol, 1 éq.) sont placés dans un ballon. Le THF anhydre est alors ajouté (9 mL, 40 mL par mmol). Le mélange réactionnel est agité à la température ambiante pendant 1h. Le solvant est en évaporé et le produit est séché sous pression réduite. Une poudre verte est obtenue (210 mg, 98%).

Le composé **Ia-K** selon la présente invention a été mis en ouvre dans des réactions d'addition 1,4 du diéthylzinc sur la 2-cyclohexènone et sur la 5-méthyl-3-hexèn-2-one.
Dans le cadre de ces exemples, le mode opératoire suivant a été utilisé.

Dans un tube de Schlenk, sont introduits une source de cuivre tel que le bis-trillate de cuivre (II) (0,02 mmol, 0,02 éq.), le composé Ia-K (0,02 mmol, 0,02 éq.), du cyclododécane (étalon interne) et un solvant anhydre (3 mL). Le mélange est agité à la température ambiante pendant 10 min avant d'ajouter le diéthylzinc (1,5 mmol, 1,5 éq.). Le milieu réactionnel est placé à température -15°C et agité pendant 5 min avant d'ajouter le substrat (1 mmol, 1 éq.).
La réaction est arrêtée par l'ajout de 5 mL d'une solution molaire d'acide chlorhydrique et le mélange est agité jusqu'à ce qu'il devienne clair. Le mélange est dilué avec de l'éther diéthylique et la phase organique est décantée, lavée avec une solution saturée de chlorure de sodium, séchée sur sulfate de magnésium, filtré et concentrée. La conversion est mesurée par chromatographie en phase gazeuse et l'excès énantiomérique est déterminé par chromatographie chirale en phase gazeuse.

Pour l'addition 1,4 du diéthylzinc sur la 2-cyclohexènone conduisant à la 3-éthylcyclohexanone, un taux de conversion de 98 % a été observé après 6 H avec un excès énantiomérique de 99 %.

### 3-éthylcyclohexanone

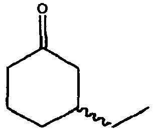

**Lipodex E, 25m ; 50°C (5 min.) - 2°C 1 min. -110°C (0 min.) -10°C 1 min. -160°C (10 min.); R_{T}(*R*) = 25,4 min. ; R_{T}(S) = 26,1 min.**

Pour l'addition 1,4 du diéthylzinc sur la 5-méthyl-3-hexen-2-one conduisant à la 4-éthyl-5-méthylhexan-2-one, un taux de conversion de 65 % a été observé après 14 H avec un excès énantiomérique de 91 %

### 4-éthyl-5-méthylhexan-2-one

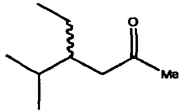

**Lipodex E, 25m ; 50°C (5min) - 0,5°C/ min -110°C (0 min.) -10°C / min. -160°C (10min.) R_{T}(*R*) = 45,3 min. ; R_{T}(S) = 47,0 min.**

Le composé **Ia-K** selon la présente invention a aussi été mis en oeuvre dans une réaction de réduction de l'acétophénone en 1-phényléthanot par transfert d'hydrures catalysée par un complexe de ruthénium RuCl₂(PPh₃)₃. Le mode opératoire suivant a été utilisé.
Dans un tube de Schlenk, sont introduits la RuCl₂(PPh₃)₃ (0,01 mmol, 0,01 éq.), le composé Ia-K selon l'invention (0,01 mmol, 0,01 éq.), le cyclododécane (étalon interne) et l'isopropanol anhydre et dégazé à froid (5 mL). Le mélange est chauffé à 80°C et agité pendant 1h. Après le refroidissement jusqu'à la température ambiante, la solution de l'acétophénone (1 mmol, 1 éq.) dans l'isopropanol anhydre (2 mL) est ajoutée. Le milieu réactionnel est réchauffé à 80°C et l'agitation est poursuivie pendant 15 min. Après refroidissement jusqu'à la température ambiante, la solution de l'hydroxyde de sodium (0,25 mmol, 0,25 éq.) dans l'isopropanol (3 mL) est ajoutée. Le milieu réactionnel est chauffé à 65°C et l'agitation est poursuivie pendant 2h.
La réaction est arrêtée par l'ajout de 0,5 mL d'une solution molaire d'acide chlorhydrique et le mélange est agité pendant 5 min. Le mélange est concentré et le résidu est repris avec 10 mL d'acétate d'éthyle et 5 mL d'eau distillée. La phase organique est décantée, lavée avec une solution saturée de chlorure de sodium, séchée sur sulfate de magnésium, filtrée et concentrée. La conversion est mesurée par chromatographie en phase gazeuse et l'excès énantiomérique est déterminé par chromatographie chirale en phase gazeuse.
Un taux de conversion de 98 % a été observé après 2h avec un excès énantiomérique de 48 %.

### 1-phénylémanol

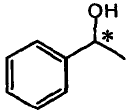

Lipodex E, 25m ; 80°C (5min) - 0,7°C/ min -130°C (0 min.) -10°C / min. -160°C (10min.) R_{T}(*R*) = 33,8 min. ; R_{T}(*S*) = 34,5 min.

## Revendications

1. Composé de formule (I): 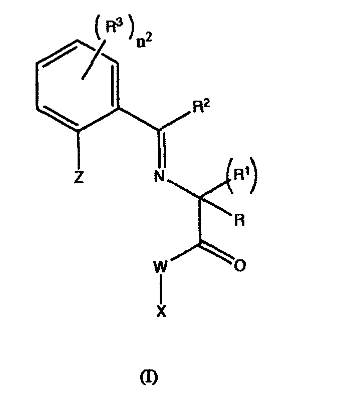 dans laquelle :
W est un atome d'oxygène ou un radical de formule NH ;
X est un hydrogène, ou un cation alcalin, ou un alkyl en C1 à C8, ou un radical - (CH₂)ₙ³- C(R⁴)(R⁵)(R⁶) où R⁴, R⁵ et R⁶ sont indépendamment un hydrogène, ou un alkyl en C1 à C9, ou un cycloalkyl en C5 ou C6 ou un aryl ou un naphtyl éventuellement substitués ;
n³ = 0 ou 1 ;
n² = 0 à 4 ;
R et R¹ sont indépendamment un alkyl en C1 à C8, ou un cycloalkyl en C5 ou C6 éventuellement substitué, ou un aryl éventuellement substitué, ou un naphtyl éventuellement substitué, ou un radical -(CH₂)ₙ⁴- C(R⁹)(R¹⁰)V où R⁹ et R¹⁰ sont indépendamment un hydrogène, ou un alkyl en C1 à C6, ou un cycloalkyl en C5 ou C6, ou un aryl ; V est un radical de formule :
(CH₂)ₙ⁵Y
Y est un hydrogène, ou un halogène, ou un OR¹¹, ou un SR¹², ou un COOR¹³, ou un NHCOR¹⁴, ou un cycloalkyl en C5 ou C6, ou un aryl éventuellement substitué ;
n⁴ = 0 ou 1 ;
n⁵ = 1 à 6 ;
R² est un hydrogène, ou alkyl en C1 à C8, ou un aryl éventuellement substitué ou un naphtyl ;
R³ est un alkyl en C1 à C6, ou un halogène, ou un OR¹⁵;
Z est un radical de formule P(R¹⁶)₂ ou un radical de forme P(O) R¹⁹:
R¹¹, R¹², R¹³, R¹⁴, R¹⁵, sont indépendamment un H, ou un alkyl en C1 à C6, ou un cycloalkyl en C5 ou C6 ou un aryl éventuellement substitué ;
R¹⁶, R¹⁷, R¹⁸, R¹⁹, R²⁰, R²¹ sont indépendamment un alkyl en C1 à C6, ou un cycloalkyl en C6, ou un aryl.

2. Composé selon la revendication 1 **caractérisé en ce qu'**il est un diphényl phosphinophényl azométhinylate.

3. Composé selon la revendication 2 **caractérisé en ce qu'**il est choisi dans le groupe constitué des composés de formules :

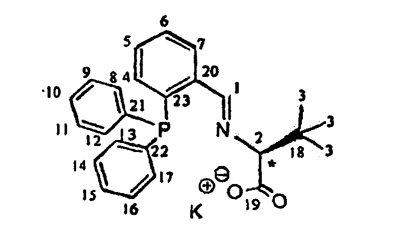

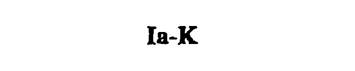

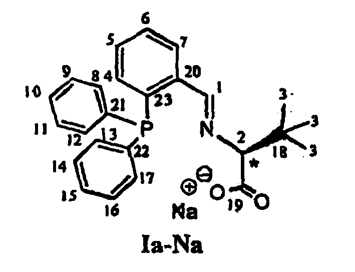

4. Composé selon la revendication 2 de formule :

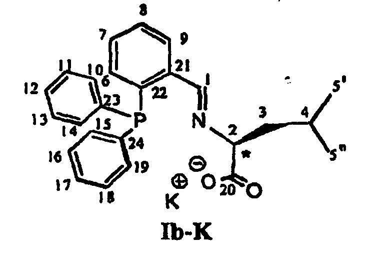

5. Complexe d'un composé selon l'une quelconque d'une revendication 1 à 4 avec au moins un métal choisi dans le groupe constitué par le cuivre, le palladium, le ruthénium, l'iridium et de rhodium.

6. Complexe selon la revendication 5 **caractérisé en ce que** ledit complexe répond à la formule :

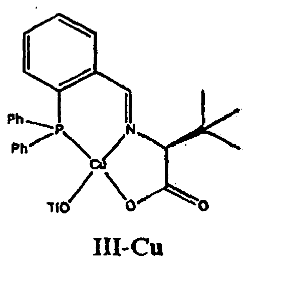

7. Procédé de synthèse d'un composé selon l'une des revendications 1 à 4 **caractérisé en ce qu'**il comprend les étapes consistant à :
obtenir un produit de formule A à partir d'un acide carboxylique aminé 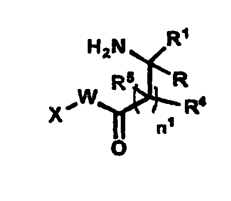
additionner au produit obtenu un aldéhyde ou une cétone choisis dans le groupe des aldéhydes ou des cétones de formule C,

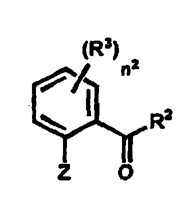

8. Procédé selon la revendication 7 **caractérisé en ce que** ledit aldéhyde est choisi dans le groupe constitué par le diphénylphosphine-benzaldéhyde et le quinoline-8 carboxaldéhyde.

9. Procédé de synthèse selon la revendication 8 d'un composé répondant à la formule Ia-K ou Ia-Na mettant en oeuvre un schéma réactionnel choisi dans le groupe des schémas réactionnels suivants :

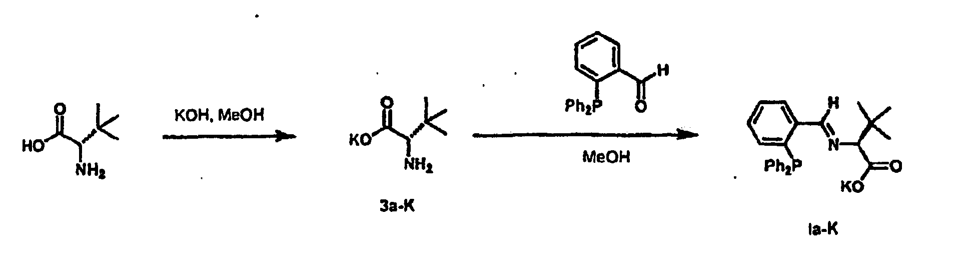

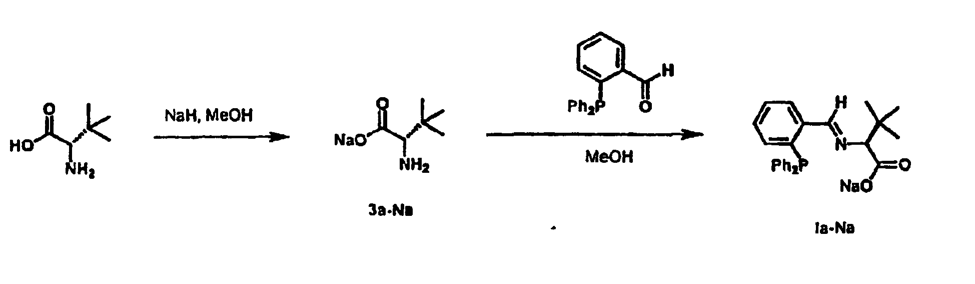

10. Utilisation d'un composé ou d'un complexe selon l'une des revendications 1 à 6 dans le cadre d'un procédé de catalyse asymétrique.

## Claims

1. Compound of the formula (I): 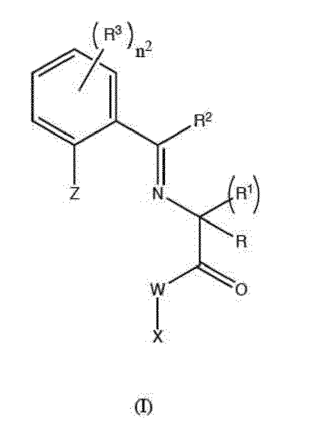 in which:
W is an oxygen atom or a radical of the formula NH;
X is a hydrogen, or an alkali metal cation, or a C1 to C8 alkyl, or a - (CH₂)ₙ³-C(R⁴) (R⁵) (R⁶) radical where R⁴, R⁵ and R⁶ independently are a hydrogen or a C1 to C9 alkyl, or a C5 or C6 cycloalkyl or an aryl or a naphthyl which are optionally substituted;
n³ = 0 or 1;
n¹ = 0;
n² = 0 to 4;
R and R¹ independently are a C1 to C8 alkyl, a C5 or C6 cycloalkyl which is optionally substituted, or an aryl which is optionally substituted, or a naphthyl which is optionally substituted, or a -(CH₂)ₙ⁴-C(R⁹) (R¹⁰)V radical where R⁹ and R¹⁰ independently are a hydrogen, or a C1 to C6 alkyl, or a C5 or C6 cycloalkyl, or an aryl; V is a radical of the formula
(CH₂) ₙ⁵Y
Y is a hydrogen, or a halogen, or an OR¹¹, or an SR¹², or a COOR¹³, or an NHCOR¹⁴, or a C5 or C6 cycloalkyl, or an aryl which is optionally substituted;
n⁴ = 0 or 1;
n⁵ = 1 to 6;
R² is a hydrogen, or a C1 to C8 alkyl, or an aryl which is optionally substituted or a naphthyl;
R³ is a C1 to C6 alkyl, or a halogen, or an OR¹⁵;
Z is a radical of the formula P(R¹⁶)₂ or a radical of the form P(O)R¹⁹;
R¹¹, R¹², R¹³, R¹⁴, R¹⁵ independently are an H, or a C1 to C6 alkyl, or a C5 or C6 cycloalkyl or an aryl which is optionally substituted;
R¹⁶, R¹⁷, R¹⁸, R¹⁹, R²⁰, R²¹ independently are a C1 to C6 alkyl, or a C6 cycloalkyl, or an aryl.

2. Compound according to claim 1, **characterized in that** it is a diphenylphosphinophenyl-azomethinylate.

3. Compound according to claim 2, **characterized in that** it is chosen from the group consisting of compounds of the formulae:

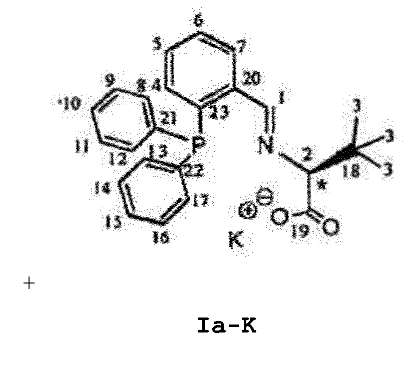

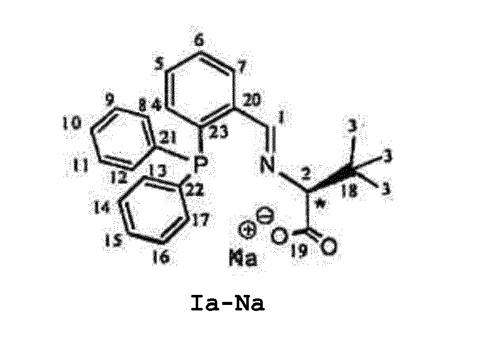

4. Compound according to claim 2, of the formula:

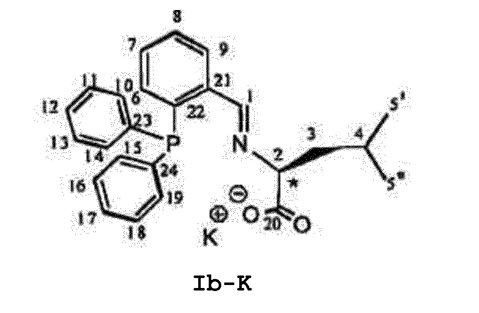

5. Complex of a compound according to any one of claims 1 to 4 with at least one metal chosen from the group consisting of copper, palladium, ruthenium, iridium and rhodium.

6. Complex according to claim 5, **characterized in that** the said complex corresponds to the formula:

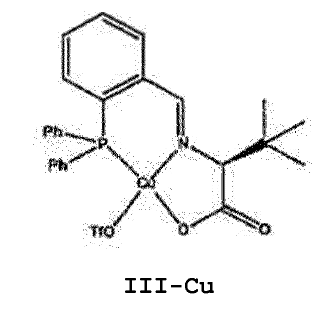

7. Process for the synthesis of a compound according to one of claims 1 to 4, **characterized in that** it comprises the stages consisting of:
obtaining a product of the formula A from an aminocarboxylic acid 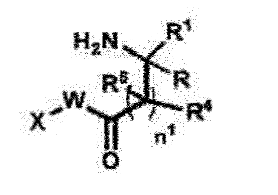
adding to the product obtained an aldehyde or a ketone chosen from the group of aldehydes or ketones of the formula C

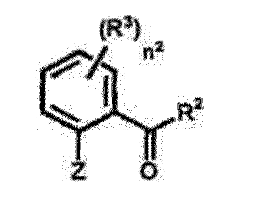

8. Process according to claim 7, **characterized in that** the said aldehyde is chosen from the group consisting of diphenylphosphine-benzaldehyde and quinoline-8-carboxaldehyde.

9. Process according to claim 8 for the synthesis of a compound corresponding to the formula Ia-K or Ia-Na implementing a reaction equation chosen from the group of the following reaction equations:

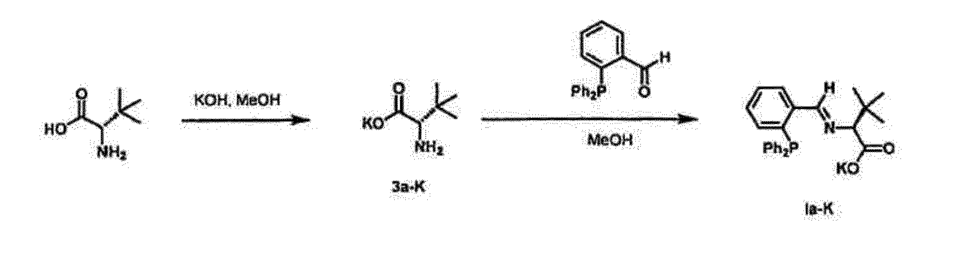

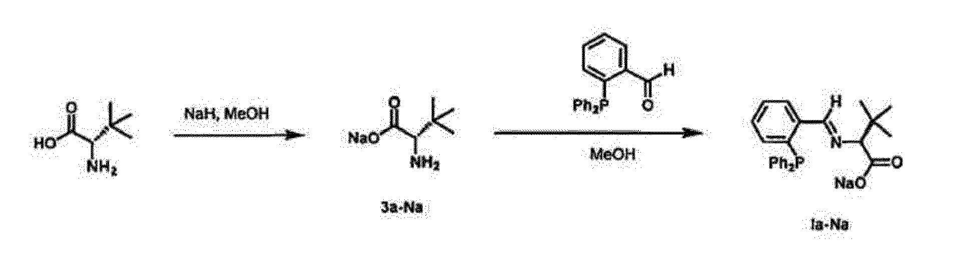

10. Use of a compound or complex according to one of claims 1 to 6 in the context of an asymmetric catalysis process.

## Patentansprüche

1. Verbindung mit der Formel (I): 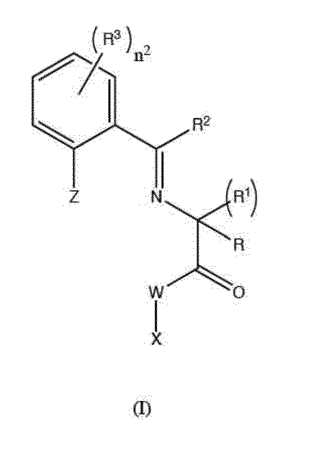 in der:
W ein Sauerstoffatom oder ein Rest mit der Formel NH ist;
X ein Wasserstoff oder ein Alkalikation oder ein C1- bis C8-Alkyl oder ein Rest - (CH₂)ₙ³-C(R⁴)(R⁵)(R⁶) ist, bei dem R⁴, R⁵ und R⁶ unabhängig ein Wasserstoff, ein C1- bis C9-Alkyl oder ein C5- oder C6-Cycloalkyl oder ein Aryl oder ein Naphthyl ist, die gegebenenfalls substituiert sind;
n³ = 0 oder 1;
n¹ = 0;
n² = 0 bis 4;
R und R¹ unabhängig ein C1- bis C8-Alkyl oder ein gegebenenfalls substituiertes C5- oder C6-Cycloalkyl, oder ein gegebenenfalls substituiertes Aryl, oder ein gegebenenfalls substituiertes Naphthyl, oder ein Rest -(CH₂)n⁴-C(R⁹)(R¹⁰)V sind, bei dem R⁹ und R¹⁰ unabhängig ein Wasserstoff oder ein C1- bis C6-Alkyl oder ein C5- oder C6-Cycloalkyl oder ein Aryl sind, wobei V ein Rest mit der Formel:
(CH₂)n⁵Y ist,
Y ein Wasserstoff oder ein Halogen oder ein OR¹¹ oder ein SR¹² oder ein COOR¹³ oder ein NHCOR¹⁴ oder ein C5- oder C6-Cycloalkyl oder ein gegebenenfalls substituiertes Aryl ist;
n⁴ = 0 oder 1;
n⁵ = 1 bis 6;
R² ein Wasserstoff oder C1- bis C8-Alkyl oder ein gegebenenfalls substituiertes Aryl oder ein Naphthyl ist;
R³ ein C1- bis C6-Alkyl oder ein Halogen oder ein OR¹⁵ ist;
Z ein Rest mit der Formel P(R¹⁶)₂ oder ein Rest mit der Formel P(O)R¹⁹ ist;
R¹¹, R¹², R¹³, R¹⁴, R¹⁵ unabhängig ein H oder ein C1- bis C6-Alkyl oder ein C5-oder C6-Cycloalkyl oder ein gegebenenfalls substituiertes Aryl sind;
R¹⁶, R¹⁷, R¹⁸, R¹⁹, R²⁰, R²¹ unabhängig ein C1- bis C6-Alkyl oder ein C6-Cycloalkyl oder ein Aryl sind.

2. Verbindung nach Anspruch 1, **dadurch gekennzeichnet, dass** sie ein Diphenylphosphinophenylazomethinylat ist.

3. Verbindung nach Anspruch 2, **dadurch gekennzeichnet, dass** sie aus der Gruppe ausgewählt ist, die aus Verbindungen mit folgenden Formeln besteht:

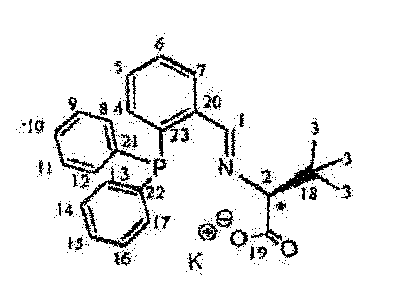

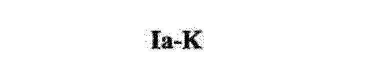

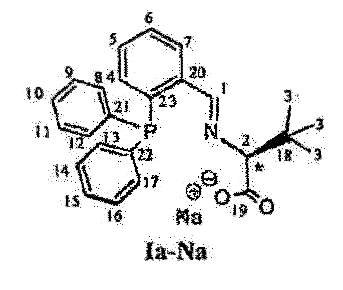

4. Verbindung nach Anspruch 2 mit folgenden Formeln:

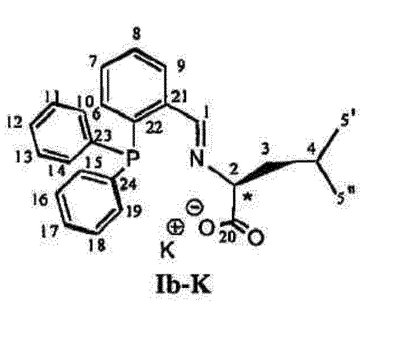

5. Komplex aus einer Verbindung nach einem der Ansprüche 1 bis 4 mit mindestens einem Metall, das aus der Gruppe ausgewählt ist, die aus Kupfer, Palladium, Ruthenium, Iridium und Rhodium besteht.

6. Komplex nach Anspruch 5, **dadurch gekennzeichnet, dass** der Komplex folgender Formel entspricht:

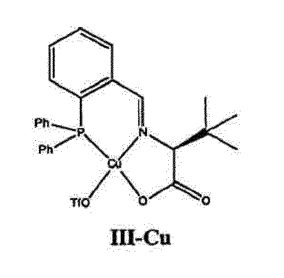

7. Verfahren zur Synthese einer Verbindung nach einem der Ansprüche 1 bis 4, **dadurch gekennzeichnet, dass** es folgende Schritte umfasst:
Gewinnen eines Produkts mit der Formel A aus einer Aminocarbonsäure 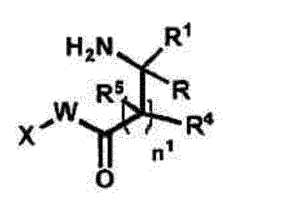
zu dem gewonnenen Produkt Zugeben eines Aldehyds oder eines Ketons, ausgewählt aus der Gruppe
der Aldehyde oder Ketone mit der Formel C

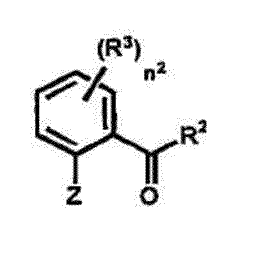

8. Verfahren nach Anspruch 7, **dadurch gekennzeichnet, dass** das Aldehyd aus der Gruppe ausgewählt ist, die aus Diphenylphosphinbenzaldehyd und Chinolin-8-carbaldehyd besteht.

9. Verfahren zur Synthese nach Anspruch 8 einer Verbindung, die der Formel la-K oder la-Na entspricht, wobei ein Reaktionsschema verwendet wird, das aus der Gruppe der folgenden Reaktionsschemata ausgewählt ist:

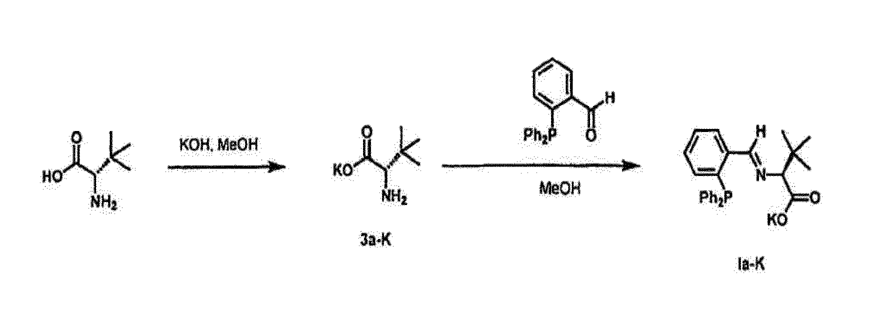

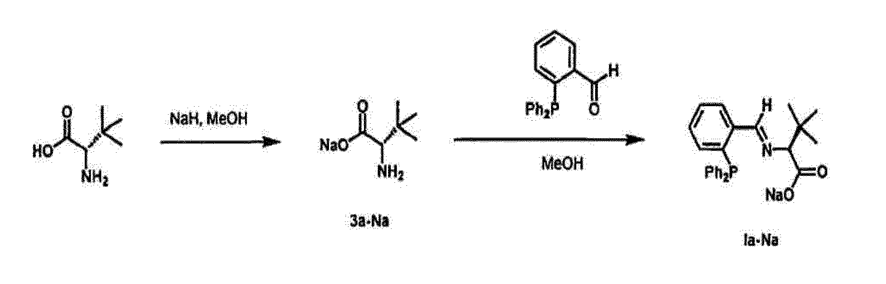

10. Verwendung einer Verbindung oder eines Komplexes nach einem der Ansprüche 1 bis 6 im Rahmen eines asymmetrischen Katalyseverfahrens.
